# EUROPÄISCHE PATENTSCHRIFT

(11) **EP 2 648 598 B1**
(45) Veröffentlichungstag und Bekanntmachung des Hinweises auf die Patenterteilung: **11.04.2018**
(21) Anmeldenummer: 11822799.0
(22) Anmeldetag: 23.11.2011
(51) Int. Cl.: A61B 1/00

(54) **HANDBETÄTIGTES ENDOSKOP FÜR MEDIZINISCHE ZWECKE**
HAND-OPERATED ENDOSCOPE FOR MEDICAL PURPOSES
ENDOSCOPE À ACTIONNEMENT MANUEL DESTINÉ À DES FINS MÉDICALES

(30) Priorität: 08.12.2010 DE 102010053814
(43) Veröffentlichungstag der Anmeldung: 16.10.2013
(73) Patentinhaber: Friedrich, Markus, 78628 Rottweil (DE)
(72) Erfinder: Friedrich, Markus, 78628 Rottweil (DE)
(74) Vertreter: Mehl-Mikus, Claudia
(86) Internationale Anmeldenummer: PCT/DE2011/002025
(87) Internationale Veröffentlichungsnummer: WO 2012/075989

(56) Entgegenhaltungen:
- WO-A1-2006/031897
- WO-A2-2008/139461
- US-A- 4 878 485
- US-A- 5 402 768

## Beschreibung

Die Erfindung betrifft ein handbetätigtes Endoskop für medizinische Zwecke nach dem Oberbegriff des Anspruchs 1.

Endoskope sind in vielfältigen Ausführungsformen bekannt. Bei einem Endoskop handelt es sich grundsätzlich um ein Gerät, mit dem das Innere von lebenden Organismen (aber auch von technischen Hohlräumen) untersucht werden kann. Insbesondere werden die Endoskope in der humanmedizinische Diagnostik sowie für Operationen eingesetzt.

Die bekannten handbetätigten Endoskope für medizinische Zwecke setzen sich aus einer Mehrzahl von Einzelgeräten zusammen. Zunächst ist eine Optik mit Okular vorgesehen. An diese Optik ist über einen Lichtleiter eine Lichtquelle angeschlossen. Weiterhin ist eine Bildkamera oder eine Videokamera vorgesehen. Diese Kamera wird über das Okular der Optik mit dieser Optik mechanisch verbunden. Schließlich ist die Kamera über ein entsprechendes Kabel mit einem Kamerasteuergerät verbunden.

Ein Nachteil bei diesem bekannten handbetätigten Endoskopiesystem besteht darin, daß es aus einer Mehrzahl von Einzelgeräten besteht. Diese sind in der Summe sehr unhandlich, was den praktischen Einsatz des Endoskops betrifft. Ein weiterer - gravierender - Nachteil besteht bei der Sterilisation des Endoskops. Die optischen sowie elektronischen Bestandteile des Endoskops sind sehr empfindlich und unterliegen wegen der notwendigen regelmäßigen Sterilisationen des Endoskops einem erhöhten Verschleiß. Dokumente US 5,402,768 und WO 2008/139461 offenbaren jeweils ein Endoskop gemäß des Oberbegriffs des Anspruchs 1. Davon ausgehend liegt der Erfindung die **Aufgabe** zugrunde, ein verbessertes handbetätigtes Endoskop für medizinische Zwecke im Hinblick auf Handlichkeit sowie im Hinblick auf verbesserte Sterilisationsmöglichkeiten zu schaffen.

Die technische **Lösung** ist gekennzeichnet durch die Merkmale im Kennzeichen des Anspruchs 1.

Ein erster Vorteil des erfindungsgemäßen handbetätigten Endoskops besteht darin, daß dieses Endoskopsystem Systeme in sich vereinigt, welche bislang nur mit verschiedenen Einzelbauteilen und Einzelgeräten realisiert werden können. Dadurch ergibt sich mit dem erfindungsgemäßen Endoskop ein überaus handliches System, das neue Operationsarbeitsweisen ermöglicht. Die Grundidee des erfindungsgemäßen Endoskops besteht in einem modularen Aufbau bestehend aus zwei Grundelementen. Zum einen ist ein - äußeres - Gehäuse vorgesehen. Zum anderen ist eine modulartige Baueinheit vorgesehen, welche in sich das Kamerasystem mit zugehöriger Optik, die Lichtquelle sowie das Übertragungssystem vereinigt. Diese modulartige Baueinheit ist so ausgebildet, daß sie dafür bestimmt und geeignet ist, als separates Teil dem Gehäuse entnommen zu werden. Zum Reinigen, Desinfizieren sowie Sterilisieren ist es lediglich notwendig, dem Gehäuse die modulartige Baueinheit zu entnehmen, um anschließend das Gehäuse für sich alleine sterilisieren zu können, ohne daß die modulartige Baueinheit mit sterilisiert werden muß. Da somit die modulartige Baueinheit mit ihren empfindlichen Teilen nicht sterilisiert wird, ergibt sich eine erheblich höhere Lebensdauer für das gesamte System. Insgesamt ist somit eine Integration der Systemkomponenten Kamera, Optik, Lichtquelle, Lichtleitung, Kameraelektronik, Stromversorgung sowie Bildübertragung in einem kompakten System geschaffen. Eine Video- und/oder Fotodokumentation ist problemlos möglich. Die Besonderheit liegt somit in der Verwendung eines sterilisierbaren, robusten äußeren Gehäuses, in welches die unsterile Baueinheit bestehend aus Kamera, Optik, Licht unter sterilen Bedingungen eingeführt werden kann. Dabei kann ein Schaft-Triokar-System mit integrierter Spül-Saug-Funktion verwendet werden.

Das erfindungsgemäße Endoskop hat vielfältige positive Auswirkungen auf die Operationstechnik. Es ist eine Reduktion der Kabelverbindungen zur Bildwiedergabe sowie zur Speichereinheit möglich, weiterhin eine verbesserte Ausleuchtung durch die Reduktion der Lichtübertragungsstrecke. Dies bedeutet insgesamt ein geringerer Aufwand sowie eine höhere Sicherheit bei der Operationsvorbereitung. Diese Operationsvorbereitung sieht so aus, daß das Gehäuse aus dem Sterilgutcontainer entnommen wird. Es wird anschließend die unsterile Baueinheit in das sterile Gehäuse eingeführt. Schließlich wird das Gehäuse dicht verschlossen. Postoperativ kann dann die Baueinheit wieder aus dem Gehäuse entnommen werden. Das demontierte Gehäuse kann somit gereinigt und einer Oberflächendesinfektion sowie -sterilisation unterzogen werden und schließlich in einem Sterilgutcontainer gelagert werden. Das Gehäuse weist einen öffenbaren sowie schließbaren Deckel auf. Dieser Deckel ist somit ein Teil des Gehäuses, welcher zusammen mit dem Gehäuse sterilisiert und desinfiziert werden kann. Der Deckel sitzt dabei dicht auf dem Gehäuse. Der Deckel kann dabei aufsteckbar oder aufschraubbar oder auf andere Art und Weise sicher mit dem Gehäuse verriegelt werden. Der Deckel kann ein bezüglich des Gehäuses separates Bauteil sein. Er kann aber auch mit dem Gehäuse verbunden sein, beispielsweise über eine Art Scharnier oder dgl.

Gemäß der Weiterbildung in Anspruch 2 ist das Gehäuse im Wesentlichen rohrförmig ausgebildet. An der Rückseite des Gehäuses befindet sich der Deckel. Dies bedeutet, daß die vorbeschriebene Baueinheit von hinten in das Gehäuse eingeführt wird. Erfindungsgemäß kann zum sterilen Einführen der Baueinheit in das Gehäuse auf die Gehäuseöffnung ein Trichter aufgesetzt werden. Der Vorteil dieses Trichters ist, daß die unsterile Baueinheit beim Einführen in das Gehäuse nicht mit der sterilen Außenseite des Gehäuses in Kontakt gelangt. Dieser Trichter wird gleichermaßen wie auch das Gehäuse sowie der Deckel vor Gebrauch sterilisiert.

Eine weitere Weiterbildung schlägt gemäß Anspruch 3 vor, daß das Gehäuse Funktionstaster besitzt. Dadurch können die elektronischen Bauteile sowie weitere Einrichtungen des Endoskops gesteuert werden. So kann der Bildausschnitt durch eine Zoomfunktion zur Verbesserung der Visualisierung direkt am Endoskop ausgewählt werden. Auch ist es möglich, die Saugfunktion bei Bedarf einhändig durch einen Taster am Gehäuse auszulösen.

Die Weiterbildung gemäß Anspruch 4 schlägt vor, daß die elektrische Kontaktierung der Baueinheit mit ihren elektronischen Bestandteilen automatisch dann erfolgt, wenn die Baueinheit in das Gehäuse eingeführt wird. Hierzu sind entsprechende Kontakte sowie korrespondierende Gegenkontakte im Innern des Gehäuses vorgesehen.

Die Übertragung der Bilddaten von dem Übertragungssystem der Baueinheit an den externen Empfänger kann gemäß der Weiterbildung in Anspruch 5 über einen entsprechenden Kabelanschluß erreicht werden. Der Kabelanschluß kontaktiert dabei mit der Übertragungseinrichtung der modulartigen Baueinheit. Hier kann eine entsprechende Steckverbindung vorgesehen sein. Diese Kabelverbindung kann auch für die Stromversorgung der elektronischen Bauteile im Innern des Gehäuses dienen.

Statt der Datenübertragung per Kabel kann auch gemäß der Weiterbildung in Anspruch 6 eine Datenübertragung (Bild- und Funktionsdaten) per Funk erfolgen. Der Vorteil besteht darin, daß dadurch ein kabelloses, handliches System geschaffen ist, welches neue Operationsarbeitsweisen ermöglicht.

Für den Fall, daß die Datenübertragung per Funk erfolgt, schlägt die Weiterbildung gemäß Anspruch 7 vor, daß die Stromversorgung für die Baueinheit mittels eines Akkumulators erfolgt. Dieser Akkumulator ist Bestandteil der vorbeschriebenen modulartigen Baueinheit.

Schließlich schlägt die Weiterbildung gemäß Anspruch 8 vor, daß die Baueinheit einen Lagesensor aufweist. Mittels dieses Lagesensors ist eine Ausrichtung des von dem Endoskop aufgenommenen Bildes auf dem Monitor möglich.

Ausführungsbeispiele eines erfindungsgemäßen handbetätigten Endoskops für medizinische Zwecke wird nachfolgend anhand der Zeichnungen beschrieben. In diesen zeigt:
- Fig. 1: eine erste Variante des Endoskops mit einer Kabelübertragung der Daten;
- Fig. 2: eine zweite Variante des Endoskops mit einer Funkübertragung der Daten;
- Fig. 3a bis 3c: der Zusammenbau des Endoskops in verschiedenen Schritten.

Das handbetätigte Endoskop besitzt ein im Wesentlichen rohrförmiges Gehäuse 1. Eine rückseitige Gehäuseöffnung wird durch einen separaten Deckel 2 dicht verschlossen. Weiterhin besitzt das Gehäuse 1 Funktionstaster 3.

Separat von dem Gehäuse 1 (mit Deckel 2) ist eine modulartige Baueinheit 4 vorgesehen. Diese besteht aus einem digitalen Kamerasystem 5 mit Optik 6 und Lichtquelle 7. Weiterhin ist ein Übertragungssystem 8 symbolisch angedeutet, welches die gewonnenen Bilddaten sowie die Steuerdaten über ein Kabel 9 einem externen Empfänger 10 übermittelt. An diesem Empfänger 10 ist ein Monitor 11 angeschlossen.

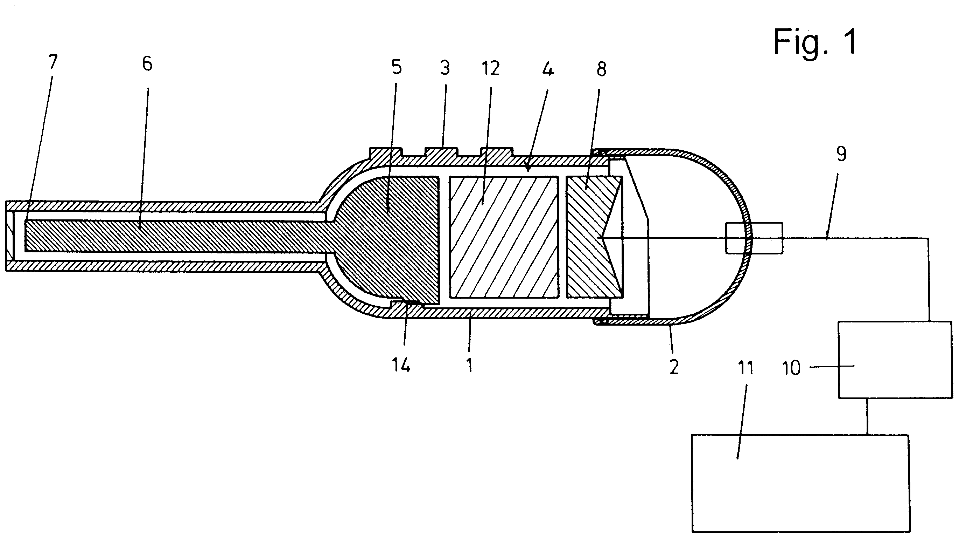

In Fig. 1 besitzt die Baueinheit 4 zusätzlich noch Akkumulator 12. Für den Fall, daß die Stromversorgung extern über ein Stromkabel erfolgt, wäre dieser Akkumulator 12 entbehrlich.

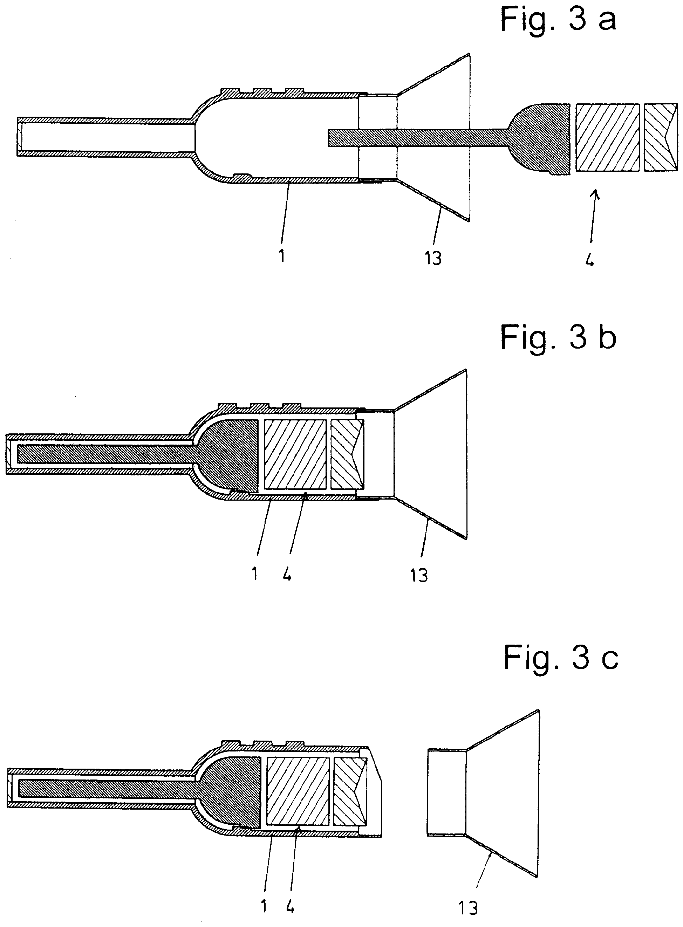

Der Zusammenbau des Endoskops wird anhand der Fig. 3a bis 3c erläutert:
Fig. 3a zeigt das sterilisierte Gehäuse 1. Auf die hintere Gehäuseöffnung ist ein ebenfalls sterilisierter Trichter 13 aufgesetzt. Dieser Trichter 13 dient als Einführhilfe zum Einfügen der unsterilen Baueinheit 4 ins Innere des Gehäuses 1.
Fig. 3b zeigt die Situation, wenn die Baueinheit 4 vollständig in das Gehäuse 1 eingeführt worden ist. Dabei kommt die Baueinheit 4 mit elektrischen Kontakten 14 des Gehäuses 1 automatisch in Kontakt. Dies bedeutet, daß über die Funktionstaster 3 Einstellungen vorgenommen werden können.
Fig. 3c zeigt die Situation, wenn der Trichter 13 vom Gehäuse 2 abgenommen worden ist und bevor schließlich der Deckel 2 auf das Gehäuse 1 aufgesetzt wird, wie dies dann in Fig. 1 dargestellt ist.

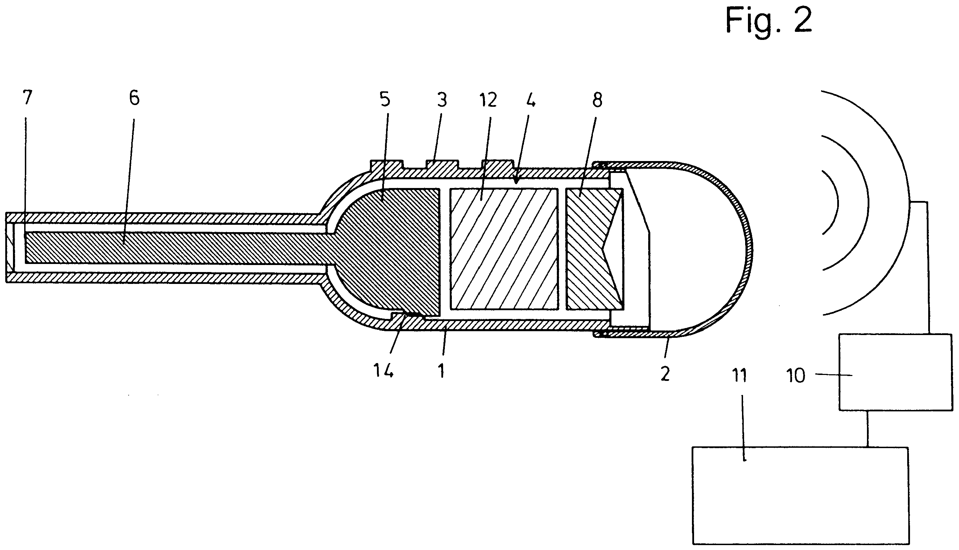

Die Ausführungsvariante in Fig. 2 unterscheidet sich von der Ausführungsvariante in Fig. 1 lediglich dadurch, daß die Datenübertragung nicht über ein Kabel 9 erfolgt, sondern daß das Übertragungssystem 8 eine Funkübertragung der Daten zu einem Funk-Empfänger 10 ermöglicht. Ansonsten unterscheidet sich diese Variante nicht von der Variante, wie sie in Fig. 1 sowie in den Fig. 3a bis 3c dargestellt ist. Allerdings ist hier bei dieser Variante in Fig. 2 zwingend ein Akkumulator 12 vorgesehen.

### Bezugszeichenliste

- 1: Gehäuse
- 2: Deckel
- 3: Funktionstaster
- 4: Baueinheit
- 5: Kamerasystem
- 6: Optik
- 7: Lichtquelle
- 8: Übertragungssystem
- 9: Kabel
- 10: Empfänger
- 11: Monitor
- 12: Akkumulator
- 13: Trichter
- 14: elektrische Kontakte

## Patentansprüche

1. Handbetätigtes Endoskop für medizinische Zwecke,
mit einem digitalen Kamerasystem (5) mit zugehöriger Optik (6),
mit einer Lichtquelle (7) sowie
mit einem Übertragungssystem (8) zum Übertragen der Bilddaten des Kamerasystems (5) oder anderer Daten zu einem externen Empfänger (10), dem ein Monitor (11) oder eine andere Visualisierungseinrichtung zur Darstellung des Bildes zugeordnet ist, wobei das Kamerasystem (5) mit zugeordneter Optik (6), die Lichtquelle (7) sowie das Übertragungssystem (8) eine modulartige Baueinheit (4) definieren und
wobei diese modulartige Baueinheit (4) als separate Baueinheit (4) in einem Gehäuse (1) mit einen öffenbaren sowie schließbaren Deckel (2) angeordnet ist,
wobei für eine Wiederverwendung des Endoskops die Baueinheit (4) komplett aus dem Gehäuse (1) herausnehmbar ist,
wobei nur das Gehäuse (1) sterilisierbar ist und wobei die unsterilisierte Baueinheit (4) oder eine andere unsterilisierte Baueinheit (4) in das sterilisierte Gehäuse (1) einfügbar ist, **dadurch gekennzeichnet,**
**daß** ein sterilisierbarer Trichter (13) vorgesehen ist, welcher bei abgenommenem Deckel (2) auf die Gehäuseöffnung zum Einführen der Baueinheit (4) in das Gehäuse (1) aufsetzbar ist.

2. Endoskop nach dem vorhergehenden Anspruch,
**dadurch gekennzeichnet,**
**daß** das Gehäuse (2) im Wesentlichen rohrförmig ausgebildet ist, wobei sich der Deckel (2) am rückwärtigen Ende des Gehäuses (1) befindet.

3. Endoskop nach einem der vorhergehenden Ansprüche,
**dadurch gekennzeichnet,**
**daß** das Gehäuse (1) Funktionstaster (3) aufweist.

4. Endoskop nach Anspruch 3,
**dadurch gekennzeichnet,**
**daß** innerhalb des Gehäuses (1) elektrische Kontakte (14) vorgesehen sind, welche in Verbindung mit den Funktionstastern (3) stehen und
welche beim Einführen der Baueinheit (4) in das Gehäuse (1) mit korrespondierenden Kontakten dieser Baueinheit (4) elektrisch kontaktieren.

5. Endoskop nach einem der Ansprüche 1 bis 4,
**dadurch gekennzeichnet,**
**daß** das Übertragungssystem (8) mit dem externen Empfänger (10) über ein Kabel (9) verbunden ist.

6. Endoskop nach einem der Ansprüche 1 bis 4,
**dadurch gekennzeichnet,**
**daß** das Übertragungssystem (8) mit dem externen Empfänger (10) über Funk verbunden ist.

7. Endoskop nach einem der vorhergehenden Ansprüche,
**dadurch gekennzeichnet,**
**daß** die Baueinheit (4) für die Stromversorgung einen Akkumulator (12) aufweist.

8. Endoskop nach einem der vorhergehenden Ansprüche,
**dadurch gekennzeichnet,**
**daß** die Baueinheit (4) einen Lagesensor aufweist.

## Claims

1. A hand-operated endoscope for medical purposes
comprising a digital camera system (5) with associated lens system (6), comprising a light source (7) as well as
comprising a data transmission system (8) for transmitting the image data of the camera system (5) or other data to an external receiver (10), to which a monitor (11) or a different visualisation device for illustrating the image, is assigned, wherein the camera system (5) with assigned lens system (6), the light source (7) a well as the transmission system (8) define a module-like component (4) and
wherein this module-like component (4) is arranged as separate component (4) in a housing (1) comprising a lid (2), which can be opened as well as closed, wherein the component (4) can be completely removed from the housing (1) for a reuse of the endoscope,
wherein only the housing (1) can be sterilised and
wherein the unsterilised component (4) or another unsterilised component (4) can be inserted into the sterilised housing (1),
**characterised in**
**that** a sterilisable funnel (13) is provided, which can be attached to the housing opening when the lid (2) is removed, for inserting the component (4) into the housing (1).

2. The endoscope according to any one of the preceding claims,
**characterised in**
**that** the housing (2) has a substantially tubular design, wherein the lid (2) is located on the rear end of the housing (1).

3. The endoscope according to any one of the preceding claims,
**characterised in**
**that** the housing (1) has function buttons (3).

4. The endoscope according to claim 3,
**characterised in**
**that** provision is made inside the housing (1) for electric contacts (14), which are connected to the function buttons (3) and which electrically contact corresponding contacts of this component (4) when the component (4) is inserted into the housing (1).

5. The endoscope according to any one of claims 1 to 4,
**characterised in**
**that** the transmission system (8) is connected to the external receiver (10) via a cable (9).

6. The endoscope according to any one of claims 1 to 4,
**characterised in**
**that** the transmission system (8) is connected to the external receiver (10) via radio.

7. The endoscope according to any one of the preceding claims,
**characterised in**
**that** the component (4) has a battery (12) for the power supply.

8. The endoscope according to any one of the preceding claims,
**characterised in**
**that** the component (4) has a position sensor.

## Revendications

1. Endoscope à actionnement manuel destiné à des fins médicales, comprenant un système de caméra numérique (5) avec une optique (6) correspondante, comprenant une source de lumière (7) ainsi qu'un système de transmission (8) pour transmettre des données d'image du système de caméra (5) ou d'autres données à un récepteur externe (10) auquel est attribué un écran (11) ou un autre dispositif de visualisation pour représenter l'image,
dans lequel le système de caméra (5) avec l'optique (6) correspondante, la source de lumière (7) ainsi que le système de transmission (8) définissent un élément modulaire (4) et
dans lequel cet élément modulaire (4) est disposé en tant qu'élément (4) séparé dans un boîtier (1) avec un couvercle (2) ouvrable et refermable,
dans lequel l'élément (4) peut être complètement sorti du boîtier (1) pour une réutilisation de l'endoscope,
dans lequel seul le boîtier (1) est stérilisable et
dans lequel l'élément (4) non stérilisé ou un autre élément (4) non stérilisé peut être inséré dans le boîtier (1) stérilisé,
**caractérisé en ce qu'**une trémie (13) stérilisable est prévue, laquelle peut être placée dans le boîtier (1) lorsque le couvercle (2) est retiré sur l'ouverture du boîtier pour introduire l'élément (4).

2. Endoscope selon la revendication précédente, **caractérisé en ce que** le boîtier (1) est conçu essentiellement tubulaire, dans lequel le couvercle (2) se trouve sur l'extrémité arrière du boîtier (1).

3. Endoscope selon l'une des revendications précédentes, **caractérisé en ce que** le boîtier (1) présente des touches fonctionnelles (3).

4. Endoscope selon la revendication 3, **caractérisé en ce que** des contacts électriques (14) sont prévus à l'intérieur du boîtier (1), lesquels sont en liaison avec les touches fonctionnelles (3) et lesquels sont en contact électrique avec des contacts correspondants de l'élément (4) lorsque cet élément (4) est introduit dans le boîtier (1).

5. Endoscope selon l'une des revendications 1 à 4, **caractérisé en ce que** le système de transmission (8) est relié au récepteur externe (10) par un câble (9).

6. Endoscope selon l'une des revendications 1 à 4, **caractérisé en ce que** le système de transmission (8) est relié au récepteur externe (10) par radio.

7. Endoscope selon l'une des revendications précédentes, **caractérisé en ce que** l'élément (4) présente un accumulateur (12) pour l'alimentation électrique.

8. Endoscope selon l'une des revendications précédentes, **caractérisé en ce que** l'élément (4) présente un capteur de position.
